(19) Europäisches Patentamt
European Patent Office
Office européen des brevets

(11) EP 1 608 674 B1

(12) **FASCICULE DE BREVET EUROPEEN**

(45) Date de publication et mention de la délivrance du brevet:
**07.12.2011 Bulletin 2011/49**

(21) Numéro de dépôt: **03816674.0**

(22) Date de dépôt: **02.04.2003**

(51) Int Cl.:
***C07K 7/06*** (2006.01) ***C07C 237/22*** (2006.01)
***C07K 5/062*** (2006.01) ***C07K 5/083*** (2006.01)
***C07K 5/103*** (2006.01)

(86) Numéro de dépôt international:
**PCT/FR2003/001021**

(87) Numéro de publication internationale:
**WO 2004/094464 (04.11.2004 Gazette 2004/45)**

(54) **OLIGOPEPTIDES, COMPOSITION ET UTILISATION COMME ELICITEURS DES DEFENSES NATURELLES DES PLANTES**

OLIGOPEPTIDE, VERBINDUNG UND VERWENDUNG DAVON ALS AUSLÖSER FÜR DIE NATÜRLCHEN ABWEHRKRÄFTE VON PFLANZEN

OLIGOPEPTIDES, COMPOSITION AND USE THEREOF AS ELICITORS OF THE NATURAL DEFENCES OF PLANTS

(84) Etats contractants désignés:
**AT BE BG CH CY CZ DE DK EE ES FI FR GB GR HU IE IT LI LU MC NL PT RO SE SI SK TR**

(43) Date de publication de la demande:
**28.12.2005 Bulletin 2005/52**

(73) Titulaires:
- **De Sangosse SA**
  **47480 Pont du Casse (FR)**
- **CENTRE NATIONAL DE LA RECHERCHE SCIENTIFIQUE (CNRS)**
  **75794 Paris Cedex 16 (FR)**

(72) Inventeurs:
- **BESNARD, Olivier**
  **F-34400 Montpellier (FR)**
- **MARTINEZ, Jean**
  **34720 Caux (FR)**
- **CAVELIER, Florine**
  **F-34170 Castelnau le Lez (FR)**

(74) Mandataire: **Fantin, Laurent et al**
**Aquinov**
**Allée de la Forestière**
**33750 Beychac et Caillau (FR)**

(56) Documents cités:
**WO-A-01/04347** **WO-A-01/98501**
**DE-A- 10 013 294**

- **CHAKRABARTTY A ET AL: "STABILITY OF ALPHA-HELICES" ADVANCES IN PROTEIN CHEMISTRY, ACADEMIC PRESS, NEW YORK, NY, US, vol. 46, 1995, pages 141-176, XP000979146 ISSN: 0065-3233**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

**Description**

L'invention concerne des oligopeptides utilisés comme éliciteurs des défenses naturelles des plantes contre les pathogènes fongiques et/ou bactériens et/ou viraux et/ou ravageurs, par application foliaire, racinaire ou par injection, et obtenus par voie de synthèse organique ou enzymatique.

Les matières actives des préparations phytosanitaires peuvent avoir une action directe sur les micro-organismes, c'est le cas des molécules inhibant certaines voies métaboliques des cellules ou qui affectent leur organisation. Il s'agit généralement d'un traitement curatif.

En lutte biologique, le traitement curatif est assuré par l'utilisation d'un agent antagoniste ou parasitaire. De même, on emploie un micro-organisme très compétitif pour la colonisation de l'espace quand il s'agit d'un traitement préventif. Les matières actives des préparations phytosanitaires peuvent avoir la propriété d'agir indirectement en activant le système de défense naturel (SDN) de la plante. Ceci se traduit par la sensibilisation de la plante à une éventuelle attaque ultérieure de la part du pathogène. A cette étape, les gènes responsables de la synthèse des protéines de défense et des phyto alexines se trouvent activés, et les produits correspondants sont synthétisés dès le premier contact entre la cellule végétale et son agresseur. On parle alors d'un traitement curatif par le biais d'éliciteurs.

Les réactions de défenses peuvent être localisées au niveau du site d'attaque, il s'agit d'une réaction d'hypersensibilité (HR) qui provoque la mort cellulaire.

Elles peuvent se généraliser et aboutir à une résistance systémique acquise (SAR). Globalement, deux mécanismes de défense concourent à freiner la propagation de la maladie. D'une part, sur le site de pénétration du pathogène, les cellules infectées s'autodétruisent pour retarder sa progression (réaction d'hypersensibilité). D'autre part, des signaux d'alertes aux cellules avoisinantes créent une zone de résistance locale acquise où s'accumulent de nombreux composés de défense. Des signaux sont également transmis à la plante entière, aboutissant ainsi à une résistance systémique acquise (SAR).

La particularité innovante de cette invention se situe au niveau du nouveau mode d'action qui est préventif. En effet, cette nouvelle classe d'éliciteurs permet de simuler une attaque de pathogènes au niveau des cellules végétales. Ces dernières vont enclencher un mécanisme naturel de défense qui va provoquer une SAR et ce, au moins une fois, ce qui permettra de prévenir encore plus efficacement l'attaque.

De très faibles quantités d'éliciteurs suffisent pour sensibiliser la membrane cytoplasmique. Ainsi, le seuil de détection des éliciteurs par les plantes peut atteindre une valeur de $10^{-9}$ Molaire ou même plus basse (Boller et al. 1995 ; Annu. Rev. Plant Physiol. Plant. Mol. Biol.).

<u>ELICITEURS</u>

Depuis l'introduction du terme éliciteur par Keen et al. (1972, Phytopathology), il a été démontré que plusieurs substances de structures chimiques diverses possèdent la propriété d'activer les systèmes naturels de défense (SDN) des plantes. Ce sont notamment ceux d'origine abiotique et qui sont représentés par les ions mercuriques, cupriques, aluminiques, les acides arachidonique, phosphorique, salycilique, fulvique et humique. Il existe aussi des éliciteurs biotiques dont les plus connus sont :

> <u>Les oligosaccharides</u> : ils font partie des premiers éliciteurs à être les mieux caractérisés (Darvill and Albersheim (1984, Annu. Rev. Plant. Physiol)]. Ils se composent de 4 classes : oligoglucannes, oligochitine, oligochitosane, oligogalacturonides, d'origine végétale [Côté et al. (1994, Plant Mol. Biol)].
> <u>Oligoglucannes</u> : l'hepta-ß-glucoside ramifié en positions (1,3-1,6) est le plus petit oligoglucoside connu pour son action élicitrice. Il a été isolé à partir de *Phytophtora sojae*.

<u>La chitine</u> : c'est un polymère linéaire de (1,4)-N-acétyl-ß-glucosamine qui se rencontre chez les champignons supérieurs et représente le constituant majeur de leurs mycella. La partie soluble de la chitine (libérée par l'action des chitinases végétales), provoque la lignification et la production de phytoalexines chez certaines plantes [Pearce et al. (1982, Physiol. Plant Pathol),Ren et al. (1992, Plant Physiol)]

<u>Les oligogalacturonides</u> : ils sont probablement libérés par la dégradation des homogalacturonanes (polysaccharides pectiques) suite à une attaque du pathogène contre la plante. Ces homogalacturonanes sont composés de résidus de l'acide 1,4-$\alpha$-D-galactosyluronique et entrent dans la composition des parois cellulaires des plantes supérieures [Côté et al. (1994, Plant Mol. Biol)].

<u>Les enzymes</u> :

Boland et al. (1997, FEBS Letters) ont démontré que le traitement de certaines plantes par une cellulase commerciale déclenche la biosynthèse de produits volatils *via* la voie de signal de l'acide octadécanoïque.

Klüsener et al. (1999, FEBS Letters) ont étudié les interactions entre des enzymes cellulolytiques d'une part, et un éliciteur issu d'une culture de levure (*Eschscholtzia californica*) d'autre part, avec des bicouches lipidiques. Ils sont arrivés à la conclusion que les éliciteurs peuvent dépolariser les membranes cytoplasmiques, influencer les flux ioniques à travers celles-ci et même perturber leurs organisations dans certains cas. Certains éliciteurs n'ont donc pas besoin de réagir avec les protéines intracellulaires pour provoquer des réponses de défense chez la plante. Ceci expliquerait le large spectre de certaines molécules.

Les Polypeptides et protéines :

Certains glycopeptides et/ou oligosaccharides libres dérivés des glycoprotéines sont actifs en terme d'élicitation [Anderson et al. (1989, ), Ebel et al. (1995, Can. J. Bot), Boller et al. (1995, Annu. Rev. Plant Physiol. Plant. Mol. Biol)]. Dans les glycoprotéines de *Colletotrichum lindemuthianum* (Coleman et al. 1992, Physiol Mol Plant Pathol) et de *Puccinia graminis f.sp. tritici* (Kogel et al. 1988, Physiol Mol Plant Pathol), les parties glucidiques sont responsables de l'activité élicitrice.

Un groupe de bactéries phytopathogènes de type Gram-négatif produit des protéines élicitrices de la HR chez des plantes non-hôtes. Par exemple, *Erwinia amylovara* (Wei et al. 1992, Science ; Baker et al. 1993, Plant Physiol) possède le gène *hrpN* qui code pour la production d'une harpin, et *Pseudomonas syringae pv.syringae* (He et al. 1993, Cell) sécrète une harpine$_{ss}$ qui est le produit du gène *hrpZ.* La protéine (*HrpN*) issue d'*Erwinia amylovora* stimule le flux extracellulaire des cations $K^+$ et régule ainsi les courants dans les cellules d'*Arabidopsis thaliana.*

Une protéine de 18 kDa sécrétée par une souche de *Trichoderma virens* a été isolée et caractérisée par Hanson et al. (2000, Phytopatology). Cette protéine est capable d'induire la biosynthèse des produits de types terpenoïdes dans le coton.

Benhamou et al. (2000, Plant physiology) décrivent qu'une protéine (oligandrin) de faible poids moléculaire, et issue de *Pythium oligandrum,* induirait une réaction de défense chez les plants de tomates. Cette réaction limiterait la progression de la maladie causée par l'agent phytopathogène *Phytophtora parasitica.*

La cryptogéine est une protéine sécrétée par le champignon *Phytophtora cryptogea* (Blein et al. 1997, FEBS Letters) qui s'en sert pour le transport du stérol. Elle est aussi connue pour ses propriétés stimulatrices de la défense des plantes (Ricci et al. 1989, Eur. J. Biochem)

Oligopeptides :

Le champignon *Phytophtora sojae* produit une glycoprotéine dont une partie (42 kDa) possède la propriété élicitrice. On a même identifié dans la moitié C-terminale de cette glycoprotéine, un oligopeptide de 13 acides aminés qui serait aussi actif [(Parker et al. 1991, Mol Plant-Microbe Interact.), (Nûmberger et al. 1994, Cell), (Sacks et al. 1995, Mol Gen Genet.)]. Une structure spécifique et une longueur minimale de la séquence sont essentielles pour que cet oligopeptide garde son activité intacte. Des phénomènes similaires ont été observés dans le cas de la systemine (Pearce et al. 1993, J. Biol Chem).

Plusieurs espèces de *Phytophtora* sécrètent des protéines extracellulaires de faible poids moléculaire (10 kDa) appelées élicitines [(Ricci et al. 1992, Plant Pathol), (Kamoun et al. 1994, Appl Environ Microbiol), (Boissy et al. 1996, Structure)]. Ces molécules sont capables de provoquer des réactions d'hypersensibilité ainsi qu'une résistance systémique acquise [(Ricci et al. 1989, Eur. J. Biochem, 183 (3)), (Kamoun et al. 1993, Mol Plant-Microbe Interact)].

Le peptide AVR9 *Cladosporium fulvum* est un éliciteurs (Wit et al. 1997, Mol Plant-Microbe Interact) de la réaction d'hypersensibilité chez les plants de tomate possédant le gène de résistance (MM-Cf9).

Acides aminés :

Siegrist et al.(2000, Physiological and Molecular Plant pathology) ont étudié la capacité de certains aminoacides à déclencher la SAR chez les plants de tabac. En travaillant avec des concentrations de 10 mM, ils ont observé les résultats suivants :

l'acide ß-aminobutyrique est actif,
l'acide $\alpha$-aminobutyrique est peu actif,
l'acide $\gamma$-aminobutyrique est totalement inactif.

SPECIFICITE DE L'INVENTION

La spécificité de l'invention repose sur l'utilisation d'Oligopeptides hélicoïdaux.

Les travaux de Boland et al. (2000, Angew. Chem. In. Ed.) ont montré que les peptaibols induisent la biosynthèse de

produits volatils chez certaines plantes. Ceci serait dû à leur capacité à former des canaux ioniques dans les membranes cellulaires. Ces types d'actions semblent être ceux qui enclencheraient la stimulation de la défense naturelle la plus forte qui soit. Ils seraient dus notamment à une nécrose localisée des cellules touchées qui induisent une alerte généralisée auprès des cellules adjacentes et des tissus.
Selon Pedras et al. (1997, Phytochemistry), la destruxine B (oligodepsipeptide cyclique) sécrétée par le champignon *Alternaria brassicae* lors de l'attaque des crucifères induirait la biosynthèse d'une phytotoxine appelée sinalexine.
Bodo et al. (1998, Biochem. et Biophys. Acta) ont étudié l'interaction des peptaibols avec les liposomes [vésicules unilamellaires larges (LUV)]. Ils ont pu montrer que le processus majeur impliqué dans l'échange ionique à travers la membrane serait le mode « tout ou rien ». La formation de pores suffisamment larges est donc essentielle pour la transition des différents ions. Ceci est assuré par la formation d'un complexe supramoléculaire entre un agrégat de 3 à 4 monomères peptidiques et les molécules lipidiques.
Yeo et al. (2000, Tetrahedon Letters) ont démontré l'activité inhibitrice des peptavirines A et B contre le virus de la mosaïque du tabac. Une inhibition de 74 à 79% a été observée en utilisant des concentrations de 10 μg/ml. Ces auteurs parlent d'un mécanisme d'action directe des peptavirines dans l'inhibition du pathogène, mais en aucun cas ils n'évoquent l'action élicitrice de ces peptaibols.

L'invention concerne l'obtention d'oligopeptides hélicoïdaux par synthèse peptidique. Les structures hélicoïdales de ces peptides se logent à l'intérieur des membranes cellulaires. Quand plusieurs de ces molécules sont insérées ensembles dans la membrane, elles forment un canal ou un pore, qui altère la perméabilité membranaire (dépolarisation). L'originalité de cette invention est donc de clarifier, optimiser et obtenir ces structures moléculaires qui permettent à coup sûr de former ces « pores ».

SYNTHESE DE POLYMERES D'AMINOACIDES

A-Polymères obtenus en mélanges

1. Obtention de polyaminoacide-alcools :

La réaction entre un aminoalcool dérivé d'un acide aminé et d'un N-Carboxyanhydride dérivé d'un aminoacide, identique $R_1$=$R_3$ et $R_2$ = $R_4$, ou différent, dans un solvant organique et sous conditions non stoechiométriques, aboutit à la formation d'un mélange d'homopolymères ou d'hétéropolymères respectivement.
Le procédé permettant l'obtention de ce type de produits peut être décrit par le schéma réactionnel suivant :

a) A partir d'α-aminoalcool et d' α-N-carboxyanhydride

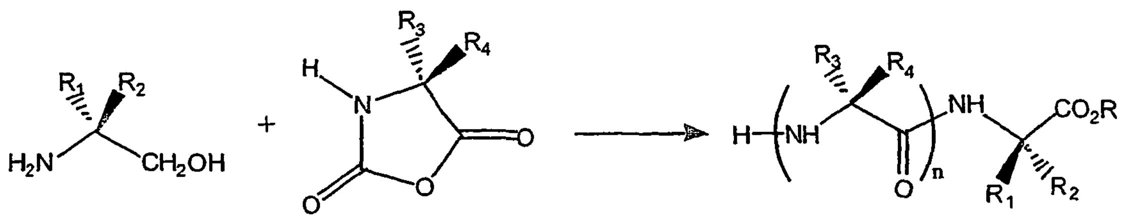

b) A partir d'α-aminoalcool, β-aminoalcool et β-N-carboxyanhydride (Cheng et al, 2000, Organic letters)

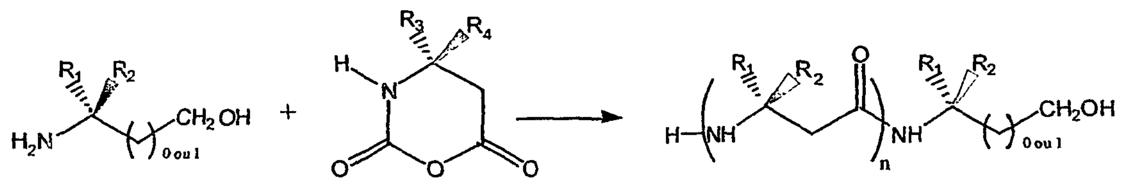

Dans les deux cas a et b : R = H, alkyl, alkyl substitué.
Selon la configuration L ou D des aminoacides utilisés :

- $R_1$ et $R_3$ = H, alkyl, alkyl substitué ou chaîne latérale des acides aminés naturels ou non naturels (protégée dans le cas de chaînes fonctionnelles) : $R_1$ et $R_3$ peuvent être identiques ;

- $R_2$ et $R_4$ = H, alkyl, alkyl substitué ou chaîne latérale des acides aminés naturels ou non naturels (protégée dans le cas de chaînes fonctionnelles) ;
- n est compris entre 3 et 30.

Procédé de synthèse :

A 20 équivalents de NCA (N-carboxyanhydride α ou β) d'aminoacide dissous dans le solvant choisi (dichlorométhane, diméthylformamide, acétonitrile, 20 ml de solvant par équivalent de NCA d'aminoacide α ou β), est ajouté 1 équivalent d'aminoalcool (α ou β). Le milieu réactionnel est agité à température ambiante pendant 6 à 48 heures, selon l'aminoacide utilisé. Le précipité obtenu est alors filtré.

Exemples :

| | | |
|---|---|---|
| Alaninol | $R_1$=$CH_3$ | $R_2$ =H |
| NCA de l'alanine: | $R_3$=CH3 | $R_4$ =H |
| NCA de l'acide glutamique | $R_3$=$CH_2CH_2COOBzl$ | $R_4$ =H |
| NCA de la valine | $R_3$=$CH(CH_3)_2$ | $R_4$ =H |

2- Obtention de polyaminoacides :

a) A partir d'α-aminoacide et d' α-N-carboxyanhydride

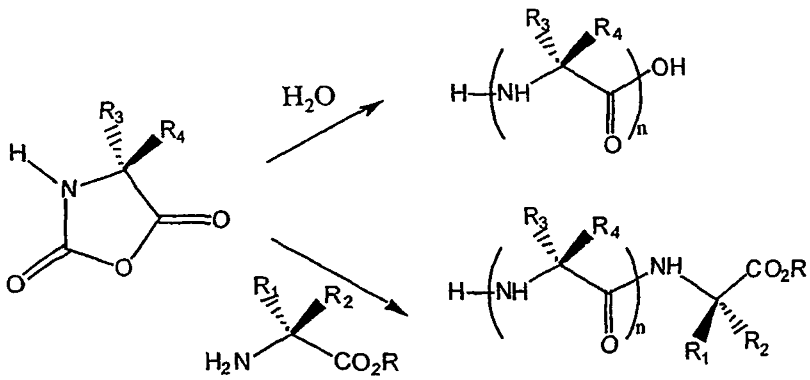


b) A partir d'α-aminoacide, β-aminoacide et β-N-carboxyanhydride

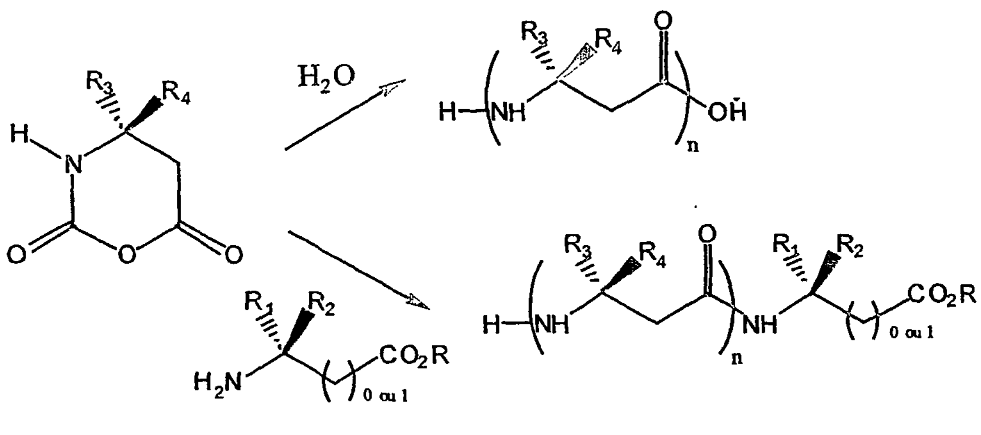

Dans les deux cas a et b : R = H, alkyl, alkyl substitué.
Selon la configuration L ou D des aminoacides utilisés :

- $R_1$ et $R_3$ = H, alkyl, alkyl substitué ou chaîne latérale des acides aminés naturels ou non naturels (protégée dans le cas de chaînes fonctionnelles). $R_1$ et $R_3$ peuvent être identiques ;
- $R_2$ et $R_4$ = H, alkyl, alkyl substitué ou chaîne latérale des acides aminés naturels ou non naturels (protégée dans le cas de chaînes fonctionnelles). $R_2$ et $R_4$ peuvent être identiques ;
- n est compris entre 3 et 30.

<u>Procédé de synthèse :</u>

A 20 équivalents de NCA (N-carboxyanhydride α ou β) d'aminoacide dissous dans le solvant adéquat (dichlorométhane, diméthylformamide, acétonitrile, 20 ml de solvant par équivalent de NCA d'aminoacide α ou β), est ajouté 1 équivalent d'eau.
La réaction est agitée à température ambiante pendant 6 à 48 heures. On filtre le précipité obtenu.

<u>Exemples :</u>

| | | |
|---|---|---|
| NCA de l'alanine : | $R_3$ =$CH_3$ | $R_4$=H |
| NCA de l'acide glutamique | $R_3$ =$CH_2CH_2COOBzl$ | $R_4$ =H |
| NCA de la valine | $R_3$ =$CH(CH_3)_2$ | $R_4$ =H |

3. <u>Obtention de polymères acylés :</u>

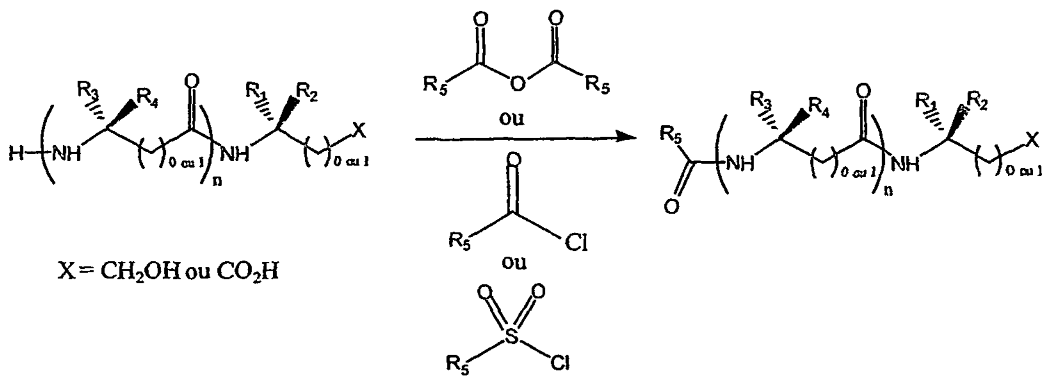


R = H, alkyl, alkyl substitué.
Selon la configuration L ou D des aminoacides utilisés :

- $R_1$ et $R_3$ = H, alkyl, alkyl substitué ou chaîne latérale des acides aminés naturels ou non naturels (protégée dans le cas de chaînes fonctionnelles). $R_1$ et $R_3$ peuvent être identiques ;
- $R_2$ et $R_4$ = H, alkyl, alkyl substitué ou chaîne latérale des acides aminés naturels ou non naturels (protégée dans le cas de chaînes fonctionnelles). $R_2$ et $R_4$ peuvent être identiques ;
- n est compris entre 3 et 30.

<u>Procédé d'acylation</u> :

250 mg de produits à acyler sont placés en suspension dans 30 ml de diméthylformamide. 15 équivalents d'agent acylant (anhydride acétique, anhydride décanoïque, etc...) sont ajoutés. La réaction est agitée à température ambiante (de 16 heures à 48 heures).
Après filtration, un solide blanc poudreux est obtenu.

B - Polymères obtenus purs et caractérisés

1. Polyaminoacide-alcools :

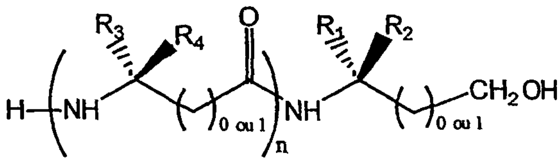

R = H, alkyl, alkyl substitué.
Selon la configuration L ou D des aminoacides utilisés :

- $R_1$ et $R_3$ = H, alkyl, alkyl substitué ou chaîne latérale des acides aminés naturels ou non naturels (protégée dans le cas de chaînes fonctionnelles). $R_1$ et $R_3$ peuvent être identiques ;
- $R_2$ et $R_4$ = H, alkyl, alkyl substitué ou chaîne latérale des acides aminés naturels ou non naturels (protégée dans le cas de chaînes fonctionnelles). $R_2$ et $R_4$ peuvent être identiques ;
- n, compris entre 3 et 20, est défini pour chaque composé.

2. Polyaminoacides :

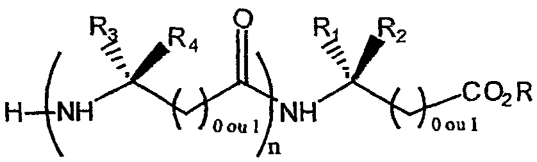

R = H, alkyl, alkyl substitué.
Selon la configuration L ou D des aminoacides utilisés :

- $R_1$ et $R_3$ = H, alkyl, alkyl substitué ou chaîne latérale des acides aminés naturels ou non naturels (protégée dans le cas de chaînes fonctionnelles). $R_1$ et $R_3$ peuvent être identiques ;
- $R_2$ et $R_4$ = H, alkyl, alkyl substitué ou chaîne latérale des acides aminés naturels ou non naturels (protégée dans le cas de chaînes fonctionnelles). $R_2$ et $R_4$ peuvent être identiques ;
- n, compris entre 3 et 20, est défini pour chaque composé.

3. Polymères acétylés :

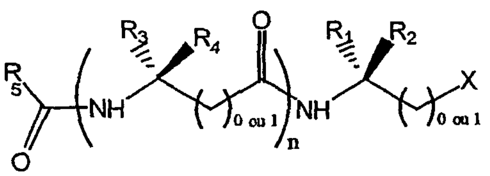

ou

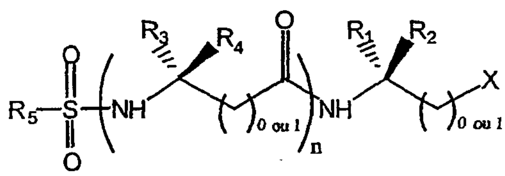

X = $CH_2OH$ ou $CO_2H$
R = H, alkyl, alkyl substitué.
Selon la configuration L ou D des aminoacides utilisés :

- $R_1$ et $R_3$ = H, alkyl, alkyl substitué ou chaîne latérale des acides aminés naturels ou non naturels (protégée dans le cas de chaînes fonctionnelles). $R_1$ et $R_3$ peuvent être identiques ;
- $R_2$ et $R_4$ = H, alkyl, alkyl substitué ou chaîne latérale des acides aminés naturels ou non naturels (protégée dans le cas de chaînes fonctionnelles). $R_2$ et $R_4$ peuvent être identiques ;
- n, compris entre 3 et 30, est défini pour chaque composé.

Exemple de procédé de synthèse des composés purs

La synthèse des composés **7, 8, 9, 10, 11, 12, 14 et 15** est réalisée en phase solide en stratégie Fmoc. La résine choisie est de type 2-chlorotrityle (Senn, 1,8 mmoles/g).
Les couplages sont effectués en présence de HOBT (2.5 équivalents) / HBTU (2.5équivalents) / DIEA (4 équivalents). Le solvant utilisé pour l'introduction des Fmoc-aminoacides est le diméthylformamide. Le groupe Fmoc est clivé par une solution de pipéridine à 20% dans le diméthylformamide.

Greffage de l'aminoalcool :

La résine est agitée à température ambiante en présence du Fmoc-aminoalcool et de 6 équivalents de pyridine dans un mélange constitué de diméthylformamide et de dichlorométhane (1/1). Après 16 h de réaction, du méthanol est additionné à la résine et le mélange réactionnel est agité pendant 30 minutes.
Après filtration, le taux de charge de la résine est déterminé par analyse UV.
Les spectres de masse ESI ont été enregistrés sur un spectromètre de masse (Micromass Platform II) en mode electrospray.

Exemple de synthèse : Composé **7**

Couplage-déprotection : A 1g de résine préchargée en alaninol (taux de substitution 0,15 $mmol.g^{-1}$) en suspension dans du DMF, est ajoutée une solution de Fmoc-Ala dans le DMF puis une solution d'activation composée d'un mélange équimolaire d'HBTU et HOBt à 0,5 M et de 4 équivalent de DIEA dans le DMF. Le mélange est agité 6 heures puis traité par une solution de pipéridine à 20% dans le DMF. La résine est lavée avec une solution de dichlorométhane puis avec une solution d'éther.
Clivage de la résine : La résine est transvasée de la vaisselle de réaction vers un tube à hémolyse auquel on a ajouté 4-5 ml d'une solution de TFA à 50% dans le dichlorométhane. Après 10 minutes sous agitation, la solution est filtrée et la résine lavée au dichlorométhane. Le solvant est évaporé sous vide.
Les composé **8** à **15** sont préparés en répétant n fois l'étape couplage-déprotection.

**1 : Ac-$Ala_n$-Ala-ol (1 < n < 10)**
ES : $[M^+H]^+$ : 261.0, 402.5, 471.7, 544.4, 615.1, 686.4, 757.3, 808.9
**2 : Ac-$Ala_n$-Ala-ol (1 < n < 5)**
ES : $[M + H]^+$ : 373.2, 444.5 ET 515.3
**3 : Ac-$Ala_n$-Ala-ol (1 < n < 8)**
ES : $[M + H]^+$ : 331.3, 402.2, 473.3, 544.6, 615.5, 685.3.
**4 : Dodécyl-$Ala_n$-Ala-ol (1 < n < 4)**
ES : $[M + H]^+$ : 328.1, 400.0, 471.8, 542.0.
**5 : Ac - $Ala_n$-Ala-ol (1 < n < 9)**
ES : $[M + H]^+$ : 189.1, 260.0, 331.1, 402.0, 472.3, 544.5, 615.7, 686.5, 757.8
**6 : H-$Ala_n$-Ala-ol (1 < n < 10)**
ES : $[M + H]^+$ : 147.0, 218.0, 288.6, 360.1, 431.1, 502.1, 573.3, 786.5.
**7 : H-$Ala_1$-Ala-ol (MW : 146)**
ES : $[M + H]^+$ : 147.0
**8 : H-$Ala_2$-Ala-ol (MW : 217)**
ES : $[M + H]^+$ : 218.0 ; $[M + NA]^+$ : 240.0 ; $[2M + H]^+$ 434.8 ; $[2M + NA]^+$ : 457.4
**9 : H-$Ala_3$-Ala-ol (MW : 288)**
ES : $[M + H]^+$ : 289.0
**10 : H-$Ala_4$-Ala-ol (MW : 359)**
ES : $[M + H]^+$ : 360.2 ; $[M + NA]^+$ : 382.2 ; $[2M + H]^+$ : 719.7 ; $[2M + NA]^+$ : 741.5
**11 : H-$Ala_5$-Ala-ol (MW : 430)**
ES : $[M + H]^+$ : 431.4 ;$[M + NA]^+$ : 453.5 ; $[2M + H]^+$ : 861.3 ; $[2M +N]^+$ : 883.8
**12 : H-$Ala_6$-Ala-OH (MW : 501)**

ES : $[M + H]^+$ ; 502.4 ; $[M + N]^+$ : 524.2

**13 : H-Ala$_n$-Ala-OH (1 < n < 10)**

ES : $[M + H]^+$ : 160.9 ; 233.8 ; 303.1 ; 374.0 ; 445.2 ; 516.2 ; 587.3 ; 658.6 ; 7293 ; 800.8

**14 : H-Ala$_7$-Ala-OH (MW : 572)**

ES : $[M + H]^+$ : 573.3 ; $[M + NA]^+$ : 595.4

**15 : H-Ala$_8$-Ala-OH (MW : 643)**

ES : $[M + H]^+$ : 644.7 ; $[M + NA]^+$ : 666.5 ; $[2M + H]^+$ : 1288.1

EFFETS PHYSIOLOGIQUES DES OLIGOPEPTIDES

1- Choix des marqueurs biochimiques sur plantes entières

a) Mesure de l'activité peroxydasique (enzyme endogène aux plantes)

Les peroxydases tiennent une place prépondérante dans les mécanismes de résistance des plantes.
Elles participent à la production d'espèces actives de l'oxygène toxiques pour les agents pathogènes et sont impliquées dans la formation de la réaction hypersensible. Elles interviennent également dans la modification de la paroi cellulaire. Il en résulte une augmentation de la synthèse de la lignine et/ou de subérine (barrières mécaniques). De même, des pontages covalents entre les protéines de la paroi cellulaire sont réalisés. Elles permettent l'oxydation de phénols en quinones très toxiques et l'incorporation des flavonoïdes dans les parois (barrière chimique).
Compte tenu du rôle primordial des peroxydases dans la résistance des plantes, nous avons utilisé l'activité peroxydasique comme marqueur de la résistance suite aux traitements par des éliciteurs.
Afin de doser les activités peroxydasiques, les feuilles sont broyées en milieu tampon citrate-monohydrogéno-phosphate-disodique. L'extrait est ensuite mis en présence du gaïacol et d'eau oxygénée. Une réaction rapide se produit : le gaïacol est transformé en tétragaïacol. L'apparition du tétragaïacol dans le milieu nous permet de calculer l'activité peroxydasique.
Les dosages sont effectués dans ce même tampon en utilisant le gaïacol comme substrat. Les résultats sont exprimés en ΔDO / mn / g de matière fraîche.

b) Mesure de l'activité chitinasique

Afin de vérifier que les mécanismes de résistance sont déclenchés, nous avons également dosé une activité enzymatique apparaissant dans les situations de résistance : l'activité chitinasique (enzyme synthétisée dès l'attaque des parasites, qui dégrade la chitine, constituant des parois des champignons phytopathogènes).
Réaction colorimétrique utilisée : on utilise un tampon acétate :

**solution de chitine + extrait enzymatique + tampon acétate qsp 0,5 ml (azur à 2 mg / ml) brut 50 mM – (pH5) (100 microlitres)**

- incubation pendant 30 minutes à 37°C, et sous agitation continue.
- on arrête la réaction par addition d'une solution d'HCl 1N
- mesure colorimétrique au spectrophotomètre à 550 nm

L'activité chitinasique est exprimée en ΔDO / mn / g de matière fraîche.

Matériel végétal utilisé

Toutes les plantes testées sont des jeunes plants obtenus par semis ou par bouturages. On pratique des pulvérisations des formulations obtenues à partir d'oligopeptides. Ces formulations contiennent divers mouillants ou pénétrants capables de véhiculer la matière active (oligopeptides) jusqu'aux cellules.

EFFETS ELICITEURS D'OLIGOPEPTIDES

Les effets éliciteurs d'oligopeptides de synthèse ont été étudiés chez plusieurs familles de plantes parmi lesquelles on peut citer : courgette, melon, concombre, salade, blé, vigne.

Cas de courgettes

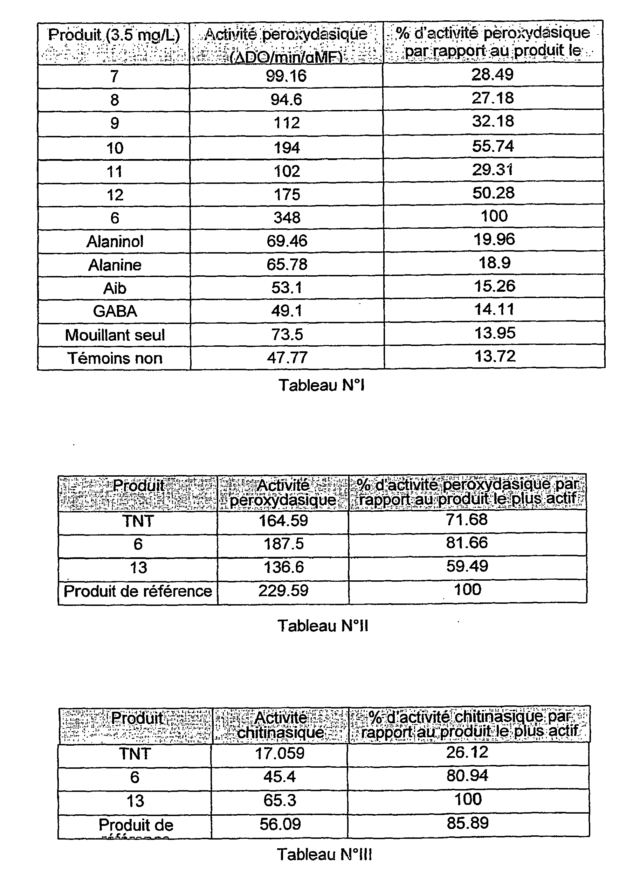

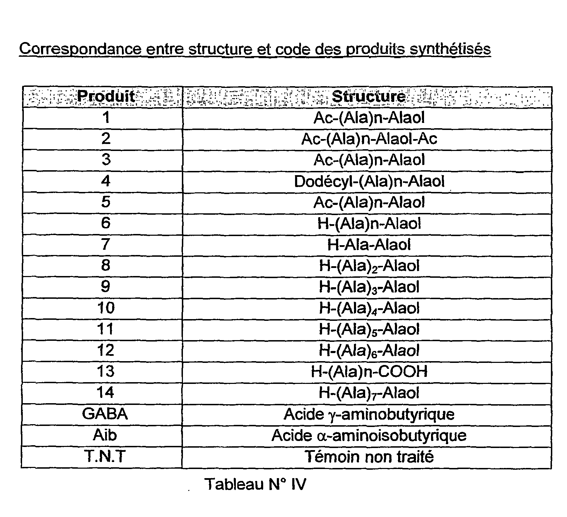

Des plants de courgette âgés de 3 semaines ont été traités par des oligopeptides. Les résultats obtenus sont résumés dans le tableau N° 1 (Planche 1/2).
D'après les résultats dudit tableau, on remarque que c'est le produit **6** qui possède la plus forte activité élicitrice.
En admettant que la valeur de 100% d'activité peroxydasique est représentée par le produit **6**, les produits **10** et **12** montrent une activité de 50%. Cette différence d'activité pourrait trouver son explication dans la diversité des structures chimiques de ces produits.
A des degrés différents, tous les autres produits ont montré une activité élicitrice. Les résultats obtenus indiquent que parmi les produits testés, les acides aminés libres sont beaucoup moins actifs par rapport aux oligopeptides. l'alaninol est le plus actif des résidus testés, suivi dans l'ordre par l'alanine, l'Aib et enfin le GABA.
Ces résultats expriment clairement le pouvoir éliciteur des oligopeptides.

Cas de la vigne

Des plants de vigne âgés de 3 semaines ont été traités par des oligopeptides. Les résultats obtenus sont résumés dans les tableaux N° II, III. (Planche 1/2)
La correspondance entre structure et code des produits synthétisés est communiqué dans le tableau N ° IV (Planche 2/2).

EFFET DE PROTECTION ANTI-PATHOGENIQUE DES OLIGOPEPTIDES

Exemple 1 : Effet contre la fusariose du melon

La pulvérisation d'oligopeptides formulés sur des plants de melon de sept jours et inoculés par *fusarium oxysporum fsp melonis* quatre jours avant, permet d'obtenir une protection contre le pathogène. Parallèlement, un lot de plantules témoins a vu ses feuilles pulvérisées avec de l'eau.
Dix jours après inoculation, les symptômes apparaissent uniquement chez les plants inoculés traités avec de l'eau. Trois semaines après l'infection, ces jeunes plants infectés se dessèchent et meurent.
Les plants inoculés et traités avec les oligopeptides ne présentent pas de symptômes avant six semaines et continuent ensuite à se développer normalement. Ces résultats expriment clairement le pouvoir éliciteur de ces oligopeptides sur la résistance des plants de melon vis-à-vis du *fusarium sp.*

Exemple 2 : Effet contre les maladies aériennes du melon

En pulvérisant des oligopeptides sur des jeunes plants de melon, on observe un effet similaire de protection contre l'*oïdium*.
Cinq jours après inoculation du pathogène, les symptômes n'apparaissent que sur les plants traités avec de l'eau. Ceux traités avec les oligopeptides ne présentent que peu ou pas de symptômes et continuent à se développer normalement trois semaines après inoculation.
Selon les caractéristiques de base de l'invention, les oligopeptides utilisés comme éliciteurs :

- sont obtenus par voie de synthèse organique ou enzymatique ;
- ont la particularité d'être des hétéro et/ou homopolymères d'acides aminés, protéiques ou non protéiques, constituant des séquences desdits polymères qui sont choisis pour leur propriété à former des structures des types hélicoïdales ou sous forme de feuillets β.

La composition selon l'invention peut :

- comporter au moins un oligopeptide comprenant au moins un acide aminé des types protéique, naturel et/ou synthétique, et/ou non protéique, naturel et/ou synthétique ;
- se présenter soit sous la forme liquide, notamment de solution aqueuse, soit sous la forme solide, notamment de poudres, granulés ou en enrobage de semences. Les oligopeptides utilisés peuvent être incorporés à un véhicule utilisé en agriculture de type mouillant et pénétrant.

L'utilisation des oligopeptides, selon l'invention, ont pour effet de réduire, lorsqu'ils sont appliqués :

- aux céréales, notamment le blé, le maïs et le riz, l'attaque des oïdiums, des septorioses, des rouilles, des fusarioses, des pyricularioses et des maladies bactériennes et virales ;

- aux arbres fruitiers, notamment le poirier et le pommier, l'attaque des didiums, tavelures, des monilioses, des maladies bactériennes et virales telles que la « Sharka » ;
- à la vigne, l'attaque de l'oïdium, du mildiou, du Botrytis, des maladies du bois, des maladies telluriques et virales telles que le « Court-Noué » ;
- aux gazons et en horticulture, les attaques des pythiacées, champignons à sclérotes, fusarioses, oïdiums, maladies bactériennes et virales ;
- aux oléagineux, notamment le soja, le tournesol, le melon, la carotte, le chou-fleur et la pomme de terre, l'attaque des oïdiums, des mildious, des pythiacées (*Phytophtora*, *Pythium*), des champignons à sclérotes (*Rhizoctonia*, *Sclerotinia*, *Pyrenocheta*), des champignons vasculaires (*Fusarium*, *Verticillium*), des maladies bactériennes et virales.

## Revendications

1. Utilisation comme éliciteur des défenses naturelles des plantes contre les pathogènes fongiques et/ou bactériens et/ou viraux et/ou ravageurs, d'un oligopeptide présentant l'une des structures suivantes :

   - Ac-$Ala_n$-Ala-ol ($1 < n < 10$)
   ES : $[M + H]^+$ ; 147.0, 218.0, 288.6, 360.1, 431.1, 502.1, 573.3, 786.5
   - H-$Ala_1$-Ala-ol
   ES : $[M + H]^+$ : 147.0
   - H-$Ala_2$-Ala-ol
   ES : $[M + H]^+$ : 218 ; $[M + Na]^+$ ; 240.0 ; $[2M + H]^+$ : 434.8 ; $[2M * Na]^+$ ; 457.4
   - H-$Ala_3$-Ala-ol
   ES : $[M + H]^+$ : 289.0
   - H-$Ala_4$-Ala-ol
   ES : $[M + H]^+$ : 260.2 ; $[M + Na]^+$ : 382.2 ; $[2M + H]^+$ : 719.7 ; $[2M + Na]^+$ : 741.5
   - H-$Ala_5$-Ala-ol
   ES : $[M + H]^+$ : 431.4 ; $[M + Na]^+$ : 453.5 ; $[2M + H]^+$ : 861.3 ; $[2M + N]^+$ : 883.8
   - H-$Ala_6$-Ala-OH
   ES : $[M + H]^+$ : 502.4 ; $[M + N]^+$ : 524.2
   - H-$Ala_n$-Ala-OH ($1 < n < 10$)
   ES : $[M + H]^+$ : 160.9 ; 233.8 ; 303.1 ; 374.0 ; 445.2 ; 516.2 ; 587.3 ; 658.6 ; 729.3 ; 800.8

2. Utilisation selon la revendication 1 pour lutter contre l'attaque des oïdiums, des septorioses, des rouilles, des fusarioses, des pyriculärioses et des maladies bactériennes et virales, sur les céréales.

3. Utilisation selon la revendication 1, pour lutter contre l'attaque des oïdiums, des tavelures, des monilioses des maladies bactériennes et virales, sur les arbres fruitiers.

4. Utilisation selon la revendication 1, pour lutter contre l'attaque des oïdiums, du mildiou, du Botrytis, des maladies du bois, des maladies telluriques et virales, sur la vigne.

5. Utilisation selon la revendication 1, pour lutter contre les attaques des pythiacées, champignons à sclérotes, des fusarioses, des oïdiums ou des maladies bactériennes et virales, sur les gazons et en horticulture.

6. Utilisation selon la revendication 1, pour lutter contre les attaques des didiums, du mildiou, des pythiacées, des champignons à sclérotes, des champignons vasculaires ou des maladies bactériennes et virales, sur les oléagineux,

## Claims

1. Use, as an elicitor of the natural defences of plants against fungal and/or bacterial and/or viral pathogens and/or pests, of an oligopeptide having one of the following structures:

   - Ac-$Ala_n$-Ala-ol ($1 < n < 10$)
   ES: $[M+H]^+$: 147.0, 218.0, 288.6, 360.1, 431.1, 502.1, 573.3, 786.5
   - H-$Ala_1$-Ala-ol

ES: $[M+H]^+$: 147.0
- H-$Ala_2$-Ala-ol
ES: $[M+H]^+$: 218; $[M+Na]^+$; 240.0; $[2M+H]^+$; 434.8; $[2M+Na]^+$; 457.4
- H-$Ala_3$-Ala-ol
ES: $[M+H]^+$: 289.0
- H-$Ala_4$-Ala-ol
ES: $[M+H]^+$: 260.2; $[M+Na]^+$; 382.2; $[2M+H]^+$; 719.7; $[2M+Na]^+$; 741.5
- H-$Ala_5$-Ala-ol
ES: $[M+H]^+$: 431.4; $[M+Na]^+$; 453.5; $[2M+H]^+$; 861.3; $[2M+Na]^+$; 883.8
- H-$Ala_6$-Ala-OH
ES: $[M+H]^+$: 502.4; $[M+N]^+$; 524.2
- H-$Ala_n$-Ala-OH (1 < n < 10)
ES: $[M+H]^+$: 160.9; 233.8; 303.1; 374.0; 445.2; 516.2; 587.3; 658.6; 729.3; 800.8

2. Use according to claim 1 for combating attacks from oidiums, septorioses, rust, fusarioses, pyricularioses, bacterial and viral diseases, on cereals.

3. Use according to claim 1, for combating attacks from oidiums, speckles, monilia diseases, bacterial and viral diseases, on fruit trees.

4. Use according to claim 1, for combating attacks from oidiums, mildew, botrytis, wood diseases, soil and viral diseases, on vines.

5. Use according to claim 1 for combating attacks from pythiaceae, sclerotium fungi, fusarioses, oidiums or bacterial and viral diseases, on lawns and in horticulture.

6. Use according to claim 1, for combating attacks from oidiums, mildew, pythiaceae, scleriotium fungi, vascular fungi or bacterial and viral diseases, on oleaginous plants.

**Patentansprüche**

1. Verwendung eines Oligopeptids als Elicitor der natürlichen Abwehr von Pflanzen gegenüber pilzlichen und/oder bakteriellen und/oder viralen Pathogenen und/oder Schädlingen, das eine der folgenden Strukturen aufweist:

- Ac-Alan-Ala-ol (1 < n < 10)
ES : $[M + H]^+$: 147.0, 218.0, 288.6, 360.1, 431.1, 502.1, 573.3, 786.5,
- H-$Ala_1$-Ala-ol
ES : $[M + H]^+$: 147.0
- H-$Ala_2$-Ala-ol
ES : $[M + H]^+$: 218; $[M + Na]^+$: 240.0; $[2M + H]^+$: 434.8; $[2M + Na]^+$: 457.4
- H-$Ala_3$-Ala-ol
ES : $[M + H]^+$: 289.0
- H-Ala4-Ala-ol
ES : $[M + H]^+$: 260.2; $[M + Na]^+$: 382.2; $[2M + H]^+$: 719.7; $[2M + Na]^+$: 741.5
- H-$Ala_5$-Ala-ol
ES : $[M + H]^+$: 431.4; $[M + Na]^+$: 453.5; $[2M + H]^+$: 861.3; $[2M + N]^+$: 883.8
- H-$Ala_6$-Ala-OH
ES : $[M + H]^+$: 502.4; $[M + N]^+$: 524.2
- H-$Ala_n$-Ala-OH (1 < n < 10)
ES : $[M + H]^+$: 160.9; 233.8; 303.1; 374.0; 445.2; 516.2; 587.3; 658.6; 729.3; 800.8

2. Verwendung nach Anspruch 1, zur Bekämpfung des Befalls mit echtem Mehltau, Septoriosen, Rostpilzen, Fusariosen, Pyricularia sowie bakteriellen und viralen Erkrankungen an Getreide.

3. Verwendung nach Anspruch 1, zur Bekämpfung des Befalls mit echtem Mehltau, Schorf, Monilia-Erkrankungen, bakteriellen und viralen Erkrankungen an Obstbäumen.

**4.** Verwendung nach Anspruch 1, zur Bekämpfung des Befalls mit echtem Mehltau, falschem Mehltau, Botrytis, Holzkrankheiten, tellurischen und viralen Erkrankungen an Weinreben.

**5.** Verwendung nach Anspruch 1, zur Bekämpfung des Befalls mit Pythiaceae, Sklerotiumpilzen, Fusariosen, echtem Mehltau oder bakteriellen und viralen Erkrankungen an Rasen und im Gartenbau.

**6.** Verwendung nach Anspruch 1, zur Bekämpfung des Befalls mit echtem Mehltau, falschem Mehltau, Pythiaceae, Sklerotiumpilzen, Gefäßpilzen oder bakteriellen und viralen Erkrankungen an Ölsaaten.

| Produit (3.5 mg/L) | Activité peroxydasique (ΔDO/min/gMF) | % d'activité peroxydasique par rapport au produit le |
|---|---|---|
| 7 | 99.16 | 28.49 |
| 8 | 94.6 | 27.18 |
| 9 | 112 | 32.18 |
| 10 | 194 | 55.74 |
| 11 | 102 | 29.31 |
| 12 | 175 | 50.28 |
| 6 | 348 | 100 |
| Alaninol | 69.46 | 19.96 |
| Alanine | 65.78 | 18.9 |
| Aib | 53.1 | 15.26 |
| GABA | 49.1 | 14.11 |
| Mouillant seul | 73.5 | 13.95 |
| Témoins non | 47.77 | 13.72 |

Tableau N°I

| Produit | Activité peroxydasique | % d'activité peroxydasique par rapport au produit le plus actif |
|---|---|---|
| TNT | 164.59 | 71.68 |
| 6 | 187.5 | 81.66 |
| 13 | 136.6 | 59.49 |
| Produit de référence | 229.59 | 100 |

Tableau N°II

| Produit | Activité chitinasique | % d'activité chitinasique par rapport au produit le plus actif |
|---|---|---|
| TNT | 17.059 | 26.12 |
| 6 | 45.4 | 80.94 |
| 13 | 65.3 | 100 |
| Produit de référence | 56.09 | 85.89 |

Tableau N°III

Correspondance entre structure et code des produits synthétisés

| Produit | Structure |
|---|---|
| 1 | Ac-(Ala)n-Alaol |
| 2 | Ac-(Ala)n-Alaol-Ac |
| 3 | Ac-(Ala)n-Alaol |
| 4 | Dodécyl-(Ala)n-Alaol |
| 5 | Ac-(Ala)n-Alaol |
| 6 | H-(Ala)n-Alaol |
| 7 | H-Ala-Alaol |
| 8 | H-$(Ala)_2$-Alaol |
| 9 | H-$(Ala)_3$-Alaol |
| 10 | H-$(Ala)_4$-Alaol |
| 11 | H-$(Ala)_5$-Alaol |
| 12 | H-$(Ala)_6$-Alaol |
| 13 | H-(Ala)n-COOH |
| 14 | H-$(Ala)_7$-Alaol |
| GABA | Acide γ-aminobutyrique |
| Aib | Acide α-aminoisobutyrique |
| T.N.T | Témoin non traité |

Tableau N° IV

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **BOLLER et al.** Annu. Rev. Plant Physiol. Plant. Mol. Biol. 1995
- **KEEN et al.** *Phytopathology,* 1972
- **DARVILL ; ALBERSHEIM.** *Annu. Rev. Plant. Physiol,* 1984
- **CÔTÉ et al.** *Plant Mol. Biol,* 1994
- **PEARCE et al.** *Physiol. Plant Pathol,* 1982
- **REN et al.** *Plant Physiol,* 1992
- **BOLAND et al.** *FEBS Letters,* 1997
- **KLÜSENER et al.** *FEBS Letters,* 1999
- **EBEL et al.** *Can. J. Bot,* 1995
- **BOLLER et al.** *Annu. Rev. Plant Physiol. Plant. Mol. Biol,* 1995
- **COLEMAN et al.** *Physiol Mol Plant Pathol,* 1992
- **KOGEL et al.** *Physiol Mol Plant Pathol,* 1988
- **WEI et al.** *Science,* 1992
- **BAKER et al.** *Plant Physiol,* 1993
- **HE et al.** *Cell,* 1993
- **HANSON et al.** *Phytopatology,* 2000
- **BENHAMOU et al.** *Plant physiology,* 2000
- **BLEIN et al.** *FEBS Letters,* 1997
- **RICCI et al.** *Eur. J. Biochem,* 1989
- **PARKER et al.** *Mol Plant-Microbe Interact,* 1991
- **NÛMBERGER et al.** *Cell,* 1994
- **SACKS et al.** *Mol Gen Genet,* 1995
- **PEARCE et al.** *J. Biol Chem,* 1993
- **RICCI et al.** *Plant Pathol,* 1992
- **KAMOUN et al.** *Appl Environ Microbiol,* 1994
- **BOISSY et al.** *Structure,* 1996
- **RICCI et al.** *Eur. J. Biochem,* 1989, vol. 183 (3
- **KAMOUN et al.** *Mol Plant-Microbe Interact,* 1993
- **WIT et al.** *Mol Plant-Microbe Interact,* 1997
- **BOLAND et al.** *Angew. Chem. In. Ed.,* 2000
- **PEDRAS et al.** *Phytochemistry,* 1997
- **BODO et al.** *Biochem. et Biophys. Acta,* 1998
- **YEO et al.** *Tetrahedon Letters,* 2000
- **CHENG et al.** *Organic letters,* 2000